# EUROPÄISCHE PATENTSCHRIFT

(11) **EP 3 362 207 B1**
(45) Veröffentlichungstag und Bekanntmachung des Hinweises auf die Patenterteilung: **05.02.2020**
(21) Anmeldenummer: 16782047.1
(22) Anmeldetag: 13.10.2016
(51) Int. Cl.: B22F 1/00, A61F 2/82, B22F 3/00, B41M 1/22

(54) **VERFAHREN ZUR HERSTELLUNG VON STENTS**
METHOD FOR PRODUCING STENTS
PROCÉDÉ DE FABRICATION D'ENDOPROTHÈSES

(30) Priorität: 15.10.2015 DE 102015220046
(43) Veröffentlichungstag der Anmeldung: 22.08.2018
(73) Patentinhaber: Fraunhofer-Gesellschaft zur Förderung der angewandten Forschung e.V., 80686 München (DE)
(72) Erfinder: QUADBECK, Peter, 01159 Dresden (DE); JURISCH, Marie, 01099 Dresden (DE); STUDNITZKY, Thomas, 01309 Dresden (DE); KIEBACK, Bernd, 01728 Bannewitz (DE)
(74) Vertreter: Pfenning, Meinig & Partner mbB
(86) Internationale Anmeldenummer: PCT/EP2016/074616
(87) Internationale Veröffentlichungsnummer: WO 2017/064201

(56) Entgegenhaltungen:
- DE-A1-102012 009 464
- US-A1- 2008 057 103
- US-A1- 2011 306 949
- US-A1- 2012 150 275

## Beschreibung

Die Erfindung betrifft ein Verfahren zur Herstellung von Stents insbesondere für die kardiovaskuläre Intervention.

Koronare Stents werden bisher durch die Strukturierung dünner Röhrchen mittels Laserbearbeitung oder Drahterodieren hergestellt. Die dünnen Röhrchen werden dabei durch vielfältiges Umformen und Rekristallisieren aus Barren gezogen. Durch die Vielzahl der Prozessschritte ist die Fertigung damit zeit- und energieaufwändig.

So sind aus US 2012/0150275 A1 ein Stent und ein Herstellungsverfahren dafür bekannt. DE 10 2012 009 464 A1 betrifft Verfahren zur Herstellung dreidimensionaler Formkörper durch schichtweisen Aufbau.

Aufgabe der Erfindung ist es daher, ein Verfahren zur Herstellung von Stents vorzuschlagen, mit dem die Herstellung von Stents einfach, schnell und kostengünstig möglich ist.

Erfindungsgemäß wird diese Aufgabe mit einem Verfahren mit den Merkmalen des Anspruchs 1 gelöst. Vorteilhafte Ausgestaltungen und Weiterbildungen können mit in untergeordneten Ansprüchen bezeichneten Merkmalen realisiert werden.

Bei einem erfindungsgemäßen Verfahren zur Herstellung von Stents insbesondere für die kardiovaskuläre Intervention wird durch ein dreidimensionales Siebdruckverfahren mit mindestens einer Suspension mindestens ein zylindrischer Hohlkörper jeweils aus einzelnen, übereinander aufgebrachten Schichten gebildet. Die Suspension ist mit einem Metallpulver und mindestens einem Binder gebildet, außerdem können Suspensionshilfsmittel enthalten sein.

Das Metallpulver kann aus den Elementen Eisen, Magnesium, Zink, Wolfram, Tantal, Titan, Niobium, Kobalt, Platin, Gold, Zirkonium oder Legierungen davon gebildet sein. Es kann so ausgewählt sein, dass ein damit hergestellter Stent bioresorbierbar ist. Das Metallpulver sollte eine Partikelgröße d₅₀ im Bereich von 0,7 µm bis 50 µm aufweisen.

Der Binder kann aus wasserlöslichen oder lösungsmittelbasierten Bindern ausgewählt sein. Suspensionshilfsmittel können Netzmittel, Verdicker oder Entschäumer sein.

Durch die Verwendung unterschiedlicher Siebe für die Ausbildung von übereinander angeordneten Schichten der Suspension können Durchbrechungen im Hohlkörper und/oder veränderte Dicken der Wandung des Hohlkörpers entlang seiner Längsachse ausgebildet werden. Mit Durchbrechungen oder Bereichen reduzierter Wandstärke kann nicht nur die Fixierung am körpereigenen Gewebe nach der Implantation verbessert werden. Diese erleichtern auch das Fließen des metallischen Werkstoffs bei der plastischen Verformung.

Strukturen und/oder Durchbrechungen in und/oder an der Wandung des Hohlkörpers können auch oder zusätzlich mittels Laserstrahl ausgebildet werden.

Anschließend werden die organischen Bestandteile der mindestens einen Suspension aus dem mindestens einen Hohlkörper ausgetrieben und der mindestens eine Hohlkörper bei einer Wärmebehandlung gesintert. Die Wärmebehandlung sollte in inerter Atmosphäre oder unter Vakuumbedingungen durchgeführt werden.

Durch das Siebdruckverfahren werden auf der Oberfläche des Hohlkörpers Überstrukturen und die einzelnen Drucklagen ausgebildet. Dies erhöht die Oberflächenrauigkeit des Hohlkörpers, wodurch die Wahrscheinlichkeit der Entstehung von Thromben im Stent erhöht wird. Daher wird die innere und/oder äußere Oberfläche des mindestens einen gesinterten Hohlkörpers durch plastische Verformung geglättet. Dies wird durch Ziehen oder Drücken des gesinterten Hohlkörpers über einen Dorn, der konisch und/oder dessen Durchmesser größer als der kleinste Innendurchmesser des gesinterten Hohlkörpers ist, und/oder durch eine Matrize mit einem Lochdurchmesser, der kleiner als der größte Außendurchmesser des gesinterten Hohlkörpers ist, erreicht.

Bei der plastischen Verformung sollte die Oberflächenrauigkeit um mindestens 30 %, bevorzugt mindestens 40 % reduziert werden.

Mit dem erfindungsgemäßen Verfahren kann mindestens ein gesinterter Hohlkörper nahtlos mit Wandstärken im Bereich von 50 µm bis 200 µm und mit Längen > 3 mm, bevorzugt > 10 mm, ausgebildet werden.

Nachfolgend soll das erfindungsgemäße Verfahren anhand eines Beispiels näher erläutert werden.

Zur Herstellung eines dünnwandigen Röhrchens wurde eine Suspension mit Edelstahl 316L als Metallpulver mit einer Partikelgröße von d₅₀ = 6 µm, Polyvinylalkohol als Binder, Wasser als Flüssigkeit und Borchi Gen NA 40 als ein mögliches Suspensionshilfsmittel gebildet. Diese wurde in 450 übereinander liegenden Schichten durch ein strukturiertes Drucksieb gerakelt.

Danach wurden die organischen Bestandteile der Suspension ausgetrieben und der zylindrische Hohlkörper bei maximalen Temperaturen bis zu 1250 °C gesintert.

Der gesinterte Hohlkörper hatte einen Durchmesser von 3,4 mm, eine Höhe von 7,4 mm und eine Wandstärke von etwa 100 µm. Die Mittenrauigkeit der Oberfläche des Hohlkörpers betrug 4,7 µm.

Der Hohlkörper wurde nun mit einem Stempel mittig fixiert und durch eine Matrize gepresst, die einen Lochdurchmesser von < 3,4 mm aufweist. Durch die dabei auftretende plastische Verformung wird die Oberfläche des Hohlkörpers geglättet. Die Mittenrauigkeit der Oberfläche des Hohlkörpers betrug danach 2,7 µm.

## Patentansprüche

1. Verfahren zur Herstellung von Stents insbesondere für die kardiovaskuläre Intervention, wobei
durch ein dreidimensionales Siebdruckverfahren mit mindestens einer Suspension, die mit einem Metallpulver und mindestens einem Binder gebildet ist, mindestens ein zylindrischer Hohlkörper jeweils aus einzelnen, übereinander aufgebrachten Schichten gebildet wird, und
anschließend organische Bestandteile der mindestens einen Suspension aus dem mindestens einen Hohlkörper ausgetrieben und
der mindestens eine Hohlkörper bei einer Wärmebehandlung gesintert wird, und
die innere und/oder äußere Oberfläche des mindestens einen gesinterten Hohlkörpers durch plastische Verformung geglättet wird; wobei
die Oberfläche eines gesinterten Hohlkörpers durch Ziehen oder Drücken des gesinterten Hohlkörpers über einen Dorn, der konisch und/oder dessen Durchmesser größer als der kleinste Innendurchmesser des gesinterten Hohlkörpers ist, und/oder durch eine Matrize mit einem Lochdurchmesser, der kleiner als der größte Außendurchmesser des gesinterten Hohlkörpers ist, geglättet wird.

2. Verfahren nach Anspruch 1, **dadurch gekennzeichnet, dass** durch die Verwendung unterschiedlicher Siebe Durchbrechungen im Hohlkörper und/oder veränderte Dicken der Wandung des Hohlkörpers entlang seiner Längsachse ausgebildet werden.

3. Verfahren nach Anspruch 1 oder 2, **dadurch gekennzeichnet, dass** Strukturen und/oder Durchbrechungen in und/oder an der Wandung des Hohlkörpers mittels Laserstrahl ausgebildet werden.

4. Verfahren nach einem der vorhergehenden Ansprüche, **dadurch gekennzeichnet, dass** das Metallpulver aus den Elementen Eisen, Magnesium, Zink, Wolfram, Tantal, Titan, Niobium, Kobalt, Platin, Gold, Zirkonium oder Legierungen davon gebildet ist.

5. Verfahren nach einem der vorhergehenden Ansprüche, **dadurch gekennzeichnet, dass** das Metallpulver eine Partikelgröße d₅₀ im Bereich von 0,7 µm bis 50 µm aufweist.

6. Verfahren nach einem der vorhergehenden Ansprüche, **dadurch gekennzeichnet, dass** die Wärmebehandlung in inerter Atmosphäre oder unter Vakuumbedingungen durchgeführt wird.

7. Verfahren nach einem der vorhergehenden Ansprüche, **dadurch gekennzeichnet, dass** mindestens ein gesinterter Hohlkörper mit Wandstärken im Bereich von 40 µm bis 400 µm ausgebildet wird.

8. Verfahren nach einem der vorhergehenden Ansprüche, **dadurch gekennzeichnet, dass** mindestens ein gesinterter Hohlkörper nahtlos mit Längen > 3 mm, bevorzugt > 10 mm, ausgebildet wird.

## Claims

1. Method for producing stents, in particular for cardiovascular intervention, wherein
at least one cylindrical hollow body is formed, in each case from individual layers applied one on top of the other, by a three-dimensional screen printing process with at least one suspension formed with a metal powder and at least one binder, and
then organic constituents of the at least one suspension are driven out of the at least one hollow body and
the at least one hollow body is sintered in a heat treatment, and
the inner and/or outer surface of the at least one sintered hollow body is smoothed by plastic deformation; wherein
the surface of a sintered hollow body is smoothed by drawing or pressing the sintered hollow body over a mandrel, which is conical and/or the diameter of which is larger than the smallest internal diameter of the sintered hollow body, and/or through a die with a hole diameter which is smaller than the largest external diameter of the sintered hollow body.

2. Method according to Claim 1, **characterized in that** the use of different screens forms apertures in the hollow body and/or varied thicknesses of the wall of the hollow body along its longitudinal axis.

3. Method according to Claim 1 or 2, **characterized in that** structures and/or apertures are formed in and/or on the wall of the hollow body by means of a laser beam.

4. Method according to one of the preceding claims, **characterized in that** the metal powder is formed from the elements iron, magnesium, zinc, tungsten, tantalum, titanium, niobium, cobalt, platinum, gold, zirconium, or alloys thereof.

5. Method according to one of the preceding claims, **characterized in that** the metal powder has a particle size d₅₀ in the range of 0.7 µm to 50 µm.

6. Method according to one of the preceding claims, **characterized in that** the heat treatment is performed in an inert atmosphere or under vacuum conditions.

7. Method according to one of the preceding claims, **characterized in that** at least one sintered hollow body is formed with wall thicknesses in the range of 40 µm to 400 µm.

8. Method according to one of the preceding claims, **characterized in that** at least one sintered hollow body is formed seamlessly with lengths of > 3 mm, preferably > 10 mm.

## Revendications

1. Procédé de fabrication de stents, en particulier pour une intervention cardiovasculaire, dans lequel
grâce à un procédé de sérigraphie tridimensionnelle utilisant au moins une suspension qui est formée d'une poudre métallique et d'au moins un liant, on forme au moins un corps creux cylindrique, constitué de couches individuelles disposées les unes au-dessus des autres, puis
on élimine de l'au moins un corps creux les constituants organiques de l'au moins une suspension, et
on fritte l'au moins un corps creux à l'aide d'un traitement thermique, et
on lisse par déformation plastique la surface intérieure et/ou la surface extérieure de l'au moins un corps creux fritté ;
la surface d'un corps creux fritté étant lissée par tirage ou compression du corps creux fritté autour d'un mandrin, qui est conique, et/ou dont le diamètre est supérieur au diamètre intérieur le plus petit du corps creux fritté, et/ou grâce à une matrice comportant un diamètre de trou qui est plus petit que le diamètre extérieur le plus grand du corps creux fritté.

2. Procédé selon la revendication 1, **caractérisé en ce que**, grâce à l'utilisation de différents tamis, on forme des ouvertures dans le corps creux et/ou des épaisseurs variables de la paroi du corps creux le long de son axe longitudinal.

3. Procédé selon la revendication 1 ou 2, **caractérisé en ce qu'**on forme des structures et/ou des ouvertures dans et/ou sur la paroi du corps creux, à l'aide d'un faisceau laser.

4. Procédé selon l'une des revendications précédentes, **caractérisé en ce que** la poudre métallique est formée des éléments fer, magnésium, zinc, tungstène, tantale, titane, niobium, cobalt, platine, or, zirconium ou des alliages de ceux-ci.

5. Procédé selon l'une des revendications précédentes, **caractérisé en ce que** la poudre métallique présente une granulométrie d₅₀ comprise dans la plage de 0,7 µm à 50 µm.

6. Procédé selon l'une des revendications précédentes, **caractérisé en ce que** le traitement thermique est mis en œuvre dans une atmosphère inerte ou dans les conditions d'un vide.

7. Procédé selon l'une des revendications précédentes, **caractérisé en ce qu'**on forme au moins un corps creux fritté ayant de épaisseurs de paroi comprises dans la plage 40 µm à 400 µm.

8. Procédé selon l'une des revendications précédentes, **caractérisé en ce qu'**on forme au moins un corps creux sans soudure, de longueurs > 3 mm, de préférence > 10 mm.
